# EUROPEAN PATENT APPLICATION

(11) **EP 0 643 076 A1**
(43) Date of publication of application: **15.03.1995**
(21) Application number: 94906370.5
(22) Date of filing: 10.02.1994
(51) Int. Cl.: C12N 15/12, C07K 13/00

(54) **NOVEL PEPTIDE HAVING RECEPTOR ACTIVITY AND DNA CODING FOR SAID PEPTIDE**

(30) Priority: 10.02.1993 JP 44330/93
(71) Applicant: TSUMURA & CO., Chuo-ku, Tokyo 103 (JP); TAKUWA, Yoh, Abikoshi, Chiba-ken 270-11 (JP)
(72) Inventor: TAKUWA, Yoh, Chiba-ken 270-11 (JP)
(74) Representative: Wain, Christopher Paul
(86) International application number: JP9400202
(87) International publication number: WO9418237

(57) **Abstract**

A novel peptide represented by SEQ ID NO 2, which binds to primary information transfer substances and has a receptor activity of conjugating with a GTP-binding protein which promotes the dissociation of GDP bound thereto; and a DNA represented by SEQ ID NO 1, which codes for the above peptide.

## Description

### Technical Field

The present invention relates to a novel peptide which has a receptor activity coupled to a GTP binding protein, and which is useful for treatment of diseases and the development of pharmaceutical products, and also relates to a DNA encoding the peptide.

### Background Art

It is known that when the vagus nerve is stimulated, heart rate becomes slower. This phenomenon is known to be caused due to the activation of a specific K⁺ channel, at a sinoatrial node, mediated by acetylcholine secreted from the nerve cells of the vagus nerve end. Acetylcholine causing such an activity is generally called a neurotransmitter.

As described above, animal cells make a function of mediating physiological responses to first messenger substances, such as neurotransmitters, hormones and autocoids, which are extracellular substances acting on the cells. Most of the first messenger substances bind to receptors which are specific glycoproteins present on the surface of a cell membrane, facing outward therefrom. A primary information transmitting substance, when binding to a receptor, activates an intracellular enzyme (hereinafter referred to as an "effecter") present in the cell membrane, facing inward therefrom. Upon activated, the intracellular enzyme produces second messenger substances, which is sent to the cell interior and mediates physiological responses specific for the second messenger substance.

It is known that in such an information transmitting system, another protein is present in a pathway from the receptor to the effecter. It has been found that a guanine nucleotide binding-regulatory protein (hereinafter referred to as a "GTP binding protein") exist in the pathway, and acts to couple the receptor activation by the first messenger to effector molecules.

The GTP binding protein exists as a complex form with a guanine nucleotide diphosphate (hereinafter referred to as "GDP"), which is generally inactive to the effector. The binding of the primary information transmitting substance to the receptor facilitates dissociation of GDP from the inactive-form GDP-GTP binding protein complex. As a result, the GDP-dissociated GTP binding protein binds to GTP and Mg⁺ions present in abundance in the cell, and is thereby converted to an active form. The active-form GTP binding protein activates the effecter.

The GTP binding protein having such a functional activity consists of three subunits, namely, α, β and γ. In the GDP-bound inactive-form of the GTP binding protein, α, β and γ are united as a complex. In the GTP-bound active form of the GTP binding protein, α is dissociated from βγ.

There have been known several types of α subunits, they are for example: (1) a type which controls an enzymatic activity concerning production of a second information transmitting substance, and (2) a type which controls the opening-shutting of a cation channel. Among the former-type subunits are: a subunit (αₛ) which activates adenylate cyclase, a subunit (αᵢ) which inactivates adenylate cyclase, a subunit (αₜ) which is present in the retina and activates cGMP phosphodiesterase, a subunit (α_{q}) which activates phospholipase C producing IP₃, and the like. Among the latter-type subunits are: a subunit (αₖ) which mediates opening a K⁺ channel, a subunit (α₀) which is present in abundance in nerve cells and mediates closing a Ca²⁺ channel, and the like.

It has been reported that functions of a βγ subunit to the effecter include (1) activation of adenylate cyclase, (2) opening K⁺ channel, (3) activation of phospholipase C, and the like.

Hundreds of types of receptors, namely, receptors coupled to the GTP binding protein have been found, which bind to the first messenger substances and facilitate dissociation of GDP from the GDP - GTP binding protein complex. As receptors coupled to GTP binding protein, known are:
Adrenaline receptor [Science, 238, 650 - 656, (1987)];
Muscarinic acetylcholine receptor [Journal of Biological Chemistry, 260, 7927 - 7935 (1985)];
Polypeptide hormone receptor [Neurokinin: Nature, 329, 836 - 838, (1987), endotheline: Nature, 348, 730 - 732 (1990)];
Thrombin receptor [Cell, 64, 1057 - 1068 (1991)]; and
Lipid hormone receptor (thromboxane A₂, etc.).

The relationship between these receptors and the GTP binding protein, particularly, a factor defining specificity of the receptors to the GTP binding protein has not yet been elucidated despite studies using peptides having a receptor activity.

Nonetheless, it is clear that the GTP binding protein and the receptors coupled to the GTP binding protein play an extremely important role in information transmission. Accordingly, to elucidate causes of physiological phenomena and diseases, and to develop pharmaceutical products, it is important to find peptides present in various organs, which have a receptor activity of coupling to the GTP binding protein, and to clarify the structure of these peptides and the corelationship between the peptides and the GTP binding protein. For that purpose, it is necessary to find a DNA encoding a peptide having a receptor activity of coupling to the GTP binding protein and to find a way of successfully cloning the peptide.

The present invention has been made in view of the above-mentioned circumstances to provide a novel peptide having a receptor activity, useful for the design of pharmaceutical products and a DNA encoding the peptide.

### Disclosure of the Invention

With the view toward newly identifying a peptide having a receptor activity of coupling to a GTP binding protein (hereinafter referred to as "a coupled-receptor"), the present inventors paid attention to the fact that there are highly homologous regions in the amino acid sequence between a known coupled receptor and other-types of known coupled receptors, and subsequently found that novel coupled-receptors can be identified by means of the polymerase chain reaction (PCR) amplification method using degenerate nucleotide mix primers corresponding to these homologous regions. Based on this finding, the present inventors have identified a novel coupled-receptor and a DNA encoding the coupled-receptor from cDNAs of rat aortic smooth muscle (RASM) cells.

More specifically, the present invention provides a novel peptide which has a receptor activity and which is encoded by a DNA containing a base sequence of SEQ ID NO. 1.

The present invention further provides a novel peptide which contains a polypeptide, as a major component, having an amino acid sequence of SEQ ID NO. 2.

The present invention still further provides a DNA which encodes a novel peptide having a receptor activity, and contains a base sequence of SEQ ID NO. 1.

Hereinbelow, the present invention will be explained in detail.

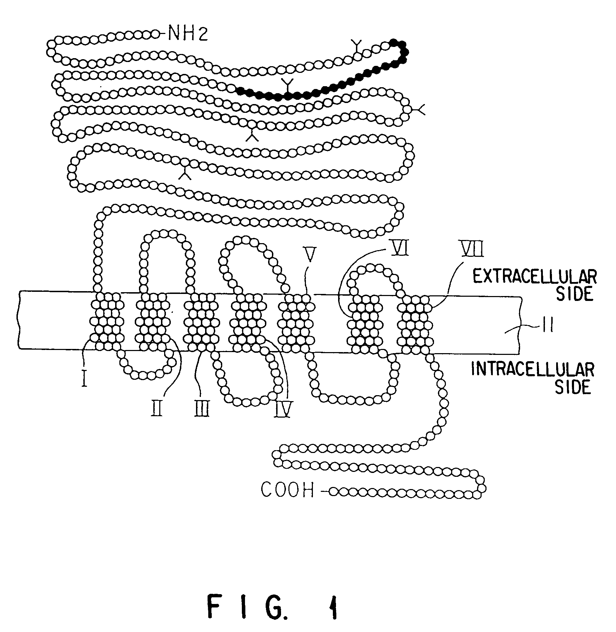

The present inventors have studied structures of amino acid sequences of known coupled-receptors and found that a coupled-receptor generally has a structure shown in FIG. 1 and is featured as follows:
(1) 7 stretches are present each consisting of hydrophobic amino acids of 20 to 26 residues, and passing through cell membrane 11 (7 stretches respectively referred to as transmembrane domains I to VII from the N-terminus).
(2) Glycosylation sites are present which link to asparagine present near the N-terminus.
(3) A cytoplasmic loop is present and presumed phosphorylated site is present near the C-terminus.

It is presumable that an unidentified novel coupled-receptor may have structural features similar to those of the coupled-receptor amino-acid sequence. Accordingly, poly (A) ⁺RNAs are isolated from the RASM cells. From the obtained poly (A) ⁺RNAs, cDNAs are synthesized. A partial cDNA corresponding to the amino acid sequence between transmembrane domains III and VI is selectively amplified by means of the PCR reaction. The selective PCR amplification method is performed using an upstream primer and a downstream primer pair whose sequences presumably correspond to the partial cDNA. As a primer, a degenerate nucleotide mix primer was actually used.

As a next step, a cDNA library derived from the RASM cells is screened using the thus-obtained PCR product as a probe, thereby obtaining a cDNA encoding the full-length of the coupled-receptor.

The coupled-receptor of the present invention can be synthesized by expressing the thus-identified DNA having a base sequence of SEQ ID NO. 1. More specifically, an appropriate DNA fragment is synthesized based on the DNA base sequence of the present invention and re-cloned into an appropriate vector such as a plasmid. This vector is then inserted into a bacteria such as Escherichia coli to obtain transformants. By expressing the DNA using the transformants, the coupled-receptor of the present invention can be obtained.

The distribution of the coupled-receptors of the present invention was determined in the following manner. After the mRNA corresponding to the DNA of the present invention was separated, in accordance with the Northern blot, from RNAs extracted from various rat tissues or cells, it was subjected to hybridization using the DNA fragment of the present invention as a probe. As a result, it was found that the coupled-receptors of the present invention are distributed predominantly in the heart, lung, stomach and intestine.

In a rat carotid-artery balloon-injury model prepared as a coronary arterial stenosis model, it was found that the coupled-receptor of the present invention relates to vascular initimal formation. More specifically, it is known that in the rat carotid-artery balloon-injury model, with facilitation of the initimal formation, vascular initima becomes thick, leading to stenosis. In vascular endotheriai cells under such conditions, the expression level of the mRNA, corresponding to the novel coupled-receptor of the present invention, is significantly lower when compared to that of normal arteries. Based on these findings, the coupled-receptor of the present invention and a DNA encoding the coupled-receptor are extremely useful for investigating causes of various diseases caused by the acceleration of the vascular initimal formation and for the design of pharmaceutical products efficient in treatment and prevention of such various diseases.

The DNA of the present invention comprises partial DNA regions corresponding to structurally characteristic sites of an amino acid sequence, which are common to the coupled-receptors such as the aforementioned transmembrane domains. Using these partial DNAs as a primer, a novel subfamily of coupled receptors can be identified.

The DNA encoding the novel peptide having the coupled-receptor activity of the present invention comprises all recombinant DNAs, chromosomes and cDNAs, which are contain the base sequence of SEQ ID NO. 1.

As explained above, according to the novel peptide which has the receptor activity of the present invention and the DNA encoding the peptides, the peptide which has a receptor activity of coupling to the GTP binding protein can be synthesized in large amounts by expressing the DNA. This peptide greatly contributes to the elucidation of the co-relationship between the GTP binding protein and receptors coupled to the GTP binding protein. Further, clarifying the amino acid sequence of the receptor coupled to the GTP binding protein and the base sequence of the DNA encoding the peptide of the receptor is not only useful for elucidating mechanisms of physiological phenomena and of diseases in connection with the receptor, but is also useful for the design of pharmaceutical products used for treatment and prevention of such diseases.

### Brief Description of the Drawings

FIG. 1 is an explanatory view showing a structure of a receptor coupled to a general GTP binding protein;
FIG. 2 is an explanatory view showing an amino acid sequence of the novel peptide having the receptor activity of the present invention and a base sequence of a DNA encoding the novel peptide;
FIG. 3 is an explanatory view showing an amino acid sequence of the novel peptide having the receptor activity of the present invention and a base sequence of a DNA encoding the novel peptide; and
FIG. 4 is an autoradiograph showing the result of the Northern blot performed in Examples in order to check the distribution of the novel peptide of the present invention.

### Best Mode of Carrying Out the Invention

### A. Determination of a base sequence of a DNA encoding a coupled-receptor

### (1) Preparation of RNA and construction of a DNA library

In this Example, RASM cells were obtained from a 5 to 10 generation-culture grown in accordance with an aortic smooth muscle tissue-slice culturing method [the explant method; Chamley-Campbell, J., Champbell, G. R., Ross, R. (1972), Physiol. Rev. 59, 1 - 61]. In accordance with the guanidine isothiocyanate/phenol/chloroform extraction method as described by Chomczynski, Sacchi, Anal. Biochem. 162, 156 - 159 (1987), a total RNA was extracted from the RASM cells. The total RNA was passed twice through a column charged with oligo dT cellulose (manufactured by Collaborative Research), thereby purifying poly (A) ⁺RNA.

Thereafter, a cDNA library was constructed in the following manner.

First, to an Eppendorf tube (1.5 ml), were placed 4 µℓ of five times-concentrated solution of a first cDNA synthesis buffer solution [250 mM Tris buffer (pH 8.3), 25 mM magnesium chloride, 375 mM potassium chloride, 50 mM dithiothreitol](manufactured by Boeringer), 2 µℓ of oligo(dT)₁₅ (manufactured by Boeringer), 6 µℓ of H₂O, 1 µℓ of an RNase inhibitor (manufactured by Boeringer), 2 µℓ of deoxynucleotides [dATP, dCTP 1 µℓ, dGTP, dTTP (manufactured by Boeringer)], [α - ³²P]dCTP, 2 µℓ (2 µg) of RASM poly (A) ⁺RNA, which had been purified in the above-mentioned manner and denatured for 3 minutes, at 70°C, and 2 µℓ of AMV reverse transcriptase (manufactured by Boeringer). The reaction mixture was incubated for 120 minutes at 42°C, thereby synthesizing a first strand of cDNA. The resultant strand, after incubated for 3 minutes at 70°C, was abruptly cooled in ice. Next, 40 µℓ of a two times-concentrated solution of a second cDNA synthesis buffer solution, 34 µℓ of H₂O, 5 µℓ of E. Coli DNA polymerase, and 1 µℓ of RNaseH were added to the Eppendorf tube, incubated for 60 minutes at 12°C, and for 60 minutes at 22°C, thereby synthesizing a second strand of the cDNA. After the enzyme was inactivated by being incubated for 10 minutes at 65°C, T4 DNA polymerase was added thereto, and incubated for 10 minutes at 37°C, thereby smoothing cDNA ends. After the resulting material was subjected to extraction with phenol/chloroform (manufactured Katayama Chemical) and ethanol precipitation, the obtained cDNA was dissolved in 3 µℓ of H₂O. To this solution were added 6 µℓ (120 pmol) of EcoRI/NotI/BamHI adaptors (manufactured by Takara Shuzo), 1 µℓ of a ten times-concentrated solution of a T4 ligase buffer solution, and 0.7 µℓ of T4 ligase (manufactured by New England BioLab), incubated for 16 hours at 15°C, thereby ligating the adaptors to both ends of the cDNA. Further, T4 ligase of the mixture was inactivated by being incubated for 15 minutes at 70°C. Thereafter, T4 polynucleotide kinase was added to the resultant solution, and incubated for 30 minutes at 37°C, thereby phosphorylating the 5' termini of the adaptors. T4 polynucleotide kinase was inactivated by being incubated for 15 minutes at 70°C, and then, subjected to chlomatography using Sephalose CL-2B (manufactured by Pharmacia Biochemical Co., Ltd), thereby selecting cDNAs having 2.0 kb or more. These cDNAs were collected by ethanol precipitation and dissolved in 9 µℓ of H₂O. Thereafter, to this solution were added 2 µg of λ gt10 phage DNA (manufactured by Stratagene) which has been digested with EcoRI and treated with alkaliphosphatase, 1 µℓ of a ten times-concentrated solution of a T4 ligase buffer solution and T4 ligase, and incubated for 16 hours at 15°C, thereby ligating the cDNA to EcoRI sites of λ gt10. To the resultant material, a λ phage extraction protein solution (manufactured by Stratagene) was added, an in-vitro packaging reaction was carried out, thereby completing the library construction. To the solution containing the thus-obtained cDNA library, were added 500 µℓ of an SM buffer [50 mM Tris buffer solution (pH 7.5), 100 mM sodium chloride, 8 mM magnesium sulfate, 0.01% gelatin] and 20 µℓ of chloroform (manufactured by Wako Pure Chemical industries Ltd.) and stored at 4°C until used.

### (2) PCR

To an Eppendorf tube were placed 8 µℓ of the five times-concentrated solution of the first cDNA synthesis buffer, 19.5 µℓ of H₂O, 2 µℓ of RNase inhibitor, 4 µℓ of deoxynucleotide, 2 µℓ of RASM poly (A) ⁺RNA which has been denatured for 3 minutes at 70°C, 0.5 µℓ of a down-stream PCR primer consisting of the base sequence of SEQ ID NO. 4, and 4 µℓ of AMV reverse transcriptase and incubated for 60 minutes at 42°C, thereby synthesizing a first cDNA strand.

To the reaction solution were added 53 µℓ of H₂O, 2 µℓ of a ten times-concentrated solution of a PCR buffer solution [100 mM Tris buffer (pH 8.3), 500 mM potassium chloride, 15 mM magnesium chloride, and 0.01% gelatin], 0.5 µℓ of an upstream primer consisting of the base sequence of SEQ ID NO. 3, and 0.5 µℓ (2.5U) of TaqDNA polymerase (manufactured by Perkin-Elmer) and subjected to a PCR. In this PCR, one cycle consists of 15 minutes at 93°C, 2 minutes at 55°C, and 4 minutes at 72°C and the cycle were repeated 25 times.

The thus-obtained PCR reactant was separated by means of electrophoresis using 1% agarose gel. A band corresponding to 300 to 600 bp was cleaved out and the PCR product was purified using the Gene Clean kit (manufactured by Bio101). After treated with restriction enzyme EcoRI (manufactured by Boeringer), the PCR product was purified using the Gene Clean Kit again and the resulting product was dissolved in 8 µℓ of H₂O. To this solution were added 20 ng of PUC118 which had been cleaved with EcoRI and treated with alkaliphosphatase, 1 µℓ of the ten times-concentrated solution of the T4 ligase buffer solution, and 0.7 µℓ of T4 ligase, and incubated for 16 hours at 15°C, thereby ligating the PCR product to PUC118.

### (3) Screening for cDNA library.

The base sequence of the PCR product sub-cloned to PUC118 in the step (2) was determined by means of the dideoxy method and identified as a PCR clone having a significant sequence homology with known coupled-receptors. The PCR clone was subjected to EcoRI treatment and a cDNA insert was cleaved out. The insert was labeled at 2 to 3 × 10⁸ cpm/µg of radio activity using [α-³²P]dCTP, randomhexaprimer, the E. Coli DNA polymerase klenow fragment (all are manufactured by Nihon Gene Co., Ltd.), thereby obtaining a probe.

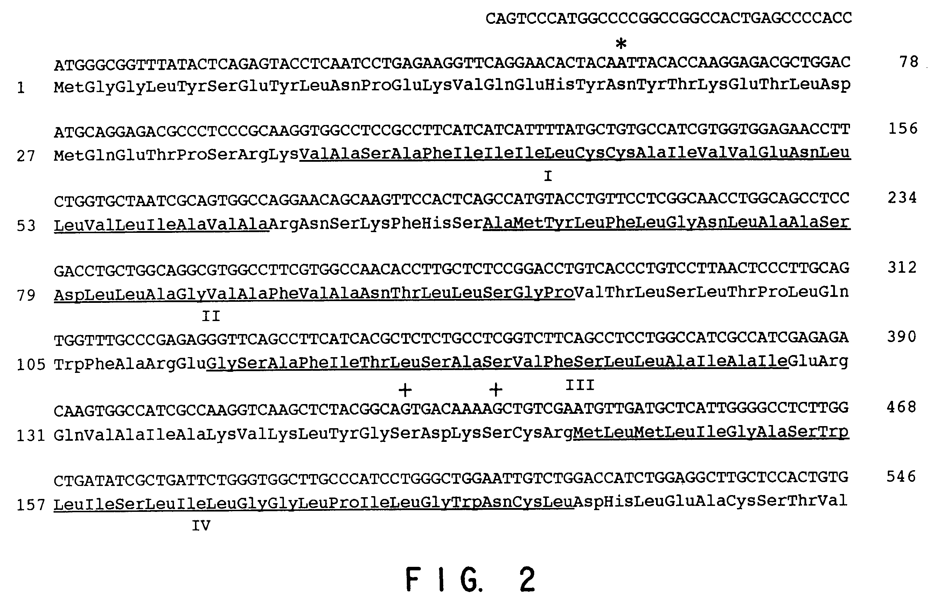

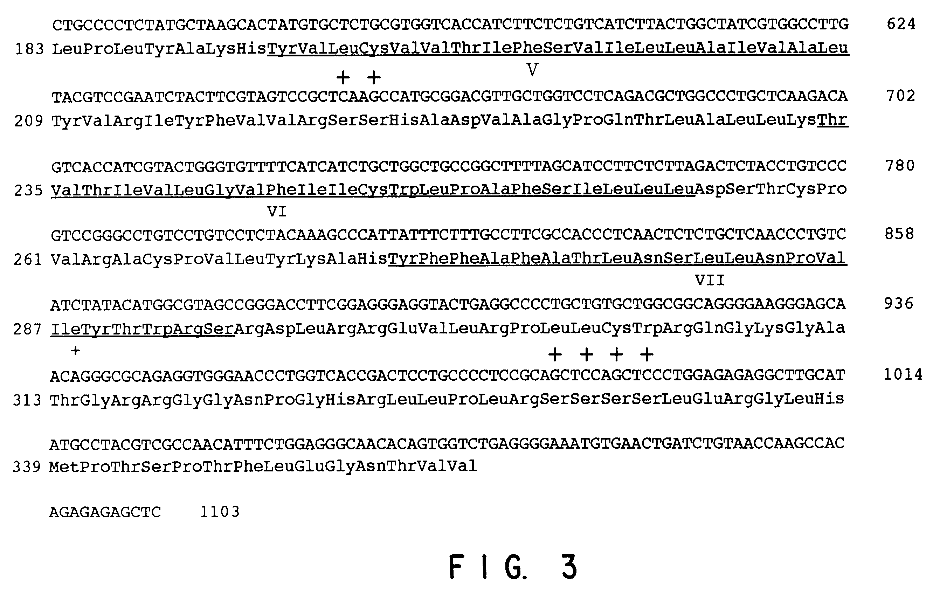

6 × 10⁵ of clones of the RASM cDNA library constructed in the step (1) were screened in accordance with the plaque hybridization method using this probe. Herein, the hybridization was performed in 0.82M sodium chloride/50% formamide/0.5% sodium dodecyl sulfate(SDS)/salmon sperm DNA (167 µg/µℓ) at 42°C. Thereafter, a filter was washed twice with a mixed solution of a two times-concentrated solution of a SSC solution (150 mM sodium chloride, 15 mM sodium citrate) and a 1% SDS at 30°C, and subjected to autoradiography. Based on this result, two positive plaques hybridized with the PCR probe were isolated. From the positive phage clones, a phage DNA was purified. From the phage DNA, an insert cDNA was cleaved out with EcoRI. The thus-obtained insert cDNA was subcloned to EcoRI sites of PUC118 in accordance with the same manner as in the subcloning of PCR product in the step (2). The base sequence of the obtained cDNA was determined in accordance with the dideoxy method. As a result, the base sequences of the coding regions of the peptides of the positive phage clones obtained from two positive plaques are completely consistent with each other. Either of the two sequences contained the full-length coding region. Among the full-length coding region, the cDNA structure of a 2.9 kb clone (hereinafter referred to as "AGR 16") are shown in FIGS. 2 and 3. The amino acid sequences of the peptides encoded by cDNA and deducted from the base sequence of the cDNA are also shown in FIGS. 2 and 3.

Mark "*" in FIGS. 2 and 3 indicates glycosylation sites linking to asparagine near the N terminus. Mark "+" indicates phosphorylation sites of a cytoplasmic loop and near the C terminus. Symbols I to VII respectively indicate transmembrane domains.

### B. Distribution of coupled receptors

In accordance with the same total RNA extraction method of the RASM cells and the same purification method of poly (A) ⁺RNA as described in the step A (1), 5 µg of poly (A) ⁺RNA or 10 µg of the total RNA obtained from the cerebrum, cerebellum, heart, lung, liver, spleen, stomach, intestine, kidney, adrenal, testis, skeletal muscle, aorta, RASM, and a rat embryonal aorta smooth muscle cell (A10) were subjected to electrophoresis using formaldehyde-added 1% agarose gel for 3 hours, at 80V. Thereafter, the RNAs were transferred from the gel to a nylon membrane. A 1.3 kb fragment from BamHI to EcoRV of the AGR 16 cDNA obtained in the step A (3) was labeled with [α - ³²P]dCTP in accordance with the same procedure as shown in the step A (3), thereby obtaining a prove.

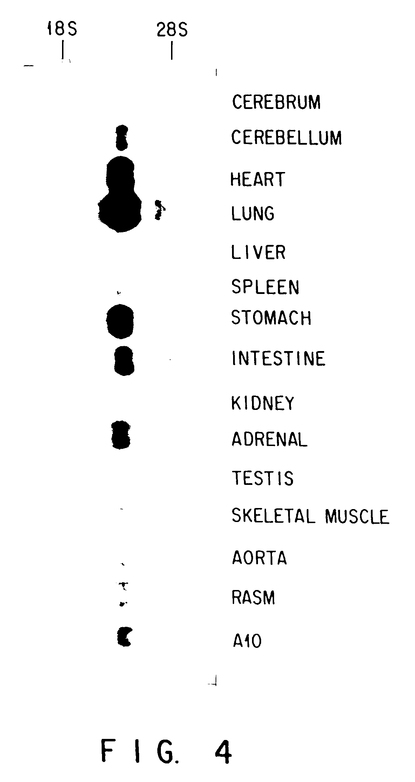

Using this probe, the Northern hybridization was performed in accordance with the same manner as in the step A (3). Thereafter, the membrane was washed with a mixed solution of 0.1SSC/0.1% SDS at 50°C, followed by subjecting to autoradiography. The results are shown in FIG. 4.

As is apparent from FIG. 4, mRNA corresponding to AGR 16 was observed to express predominantly in the heart, lung, stomach, and intestine.

SEQUENCE LISTINGS
SEQ ID NO: 1
LENGTH OF SEQUENCE: 1139
TYPE OF SEQUENCE : nucleic acid
STRANDEDNESS : single
TOPOLOGY : linear
TYPE OF SEQUENCE : other nucleic acid synthetic DNA
SEQUENCE DESCRIPTION
CAGTCCCATG GCCCCGGCCG GCCACTGAGC CCCACCATGG GCGGTTTATA 50
CTCAGAGTAC CTCAATCCTG AGAAGGTTCA GGAACACTAC AATTACACCA 100
AGGAGACGCT GGACATGCAG GAGACGCCCT CCCGCAAGGT GGCCTCCGCC 150
TTCATCATCA TTTTATGCTG TGCCATCGTG GTGGAGAACC TTCTGGTGCT 200
AATCGCAGTG GCCAGGAACA GCAAGTTCCA CTCAGCCATG TACCTGTTCC 250
TCGGCAACCT GGCAGCCTCC GACCTGCTGG CAGGCGTGGC CTTCGTGGCC 300
AACACCTTGC TCTCCGGACC TGTCACCCTG TCCTTAACTC CCTTGCAGTG 350
GTTTGCCCGA GAGGGTTCAG CCTTCATCAC GCTCTCTGCC TCGGTCTTCA 400
GCCTCCTGGC CATCGCCATC GAGAGACAAG TGGCCATCGC CAAGGTCAAG 450
CTCTACGGCA GTGACAAAAG CTGTCGAATG TTGATGCTCA TTGGGGCCTC 500
TTGGCTGATA TCGCTGATTC TGGGTGGCTT GCCCATCCTG GGCTGGAATT 550
GTCTGGACCA TCTGGAGGCT TGCTCCACTG TGCTGCCCCT CTATGCTAAG 600
CACTATGTGC TCTGCGTGGT CACCATCTTC TCTGTCATCT TACTGGCTAT 650
CGTGGCCTTG TACGTCCGAA TCTACTTCGT AGTCCGCTCA AGCCATGCGG 700
ACGTTGCTGG TCCTCAGACG CTGGCCCTGC TCAAGACAGT CACCATCGTA 750
CTGGGTGTTT TCATCATCTG CTGGCTGCCG GCTTTTAGCA TCCTTCTCTT 800
AGACTCTACC TGTCCCGTCC GGGCCTGTCC TGTCCTCTAC AAAGCCCATT 850
ATTTCTTTGC CTTCGCCACC CTCAACTCTC TGCTCAACCC TGTCATCTAT 900
ACATGGCGTA GCCGGGACCT TCGGAGGGAG GTACTGAGGC CCCTGCTGTG 950

CTGGCGGCAG GGGAAGGGAG CAACAGGGCG CAGAGGTGGG AACCCTGGTC 1000
ACCGACTCCT GCCCCTCCGC AGCTCCAGCT CCCTGGAGAG AGGCTTGCAT 1050
ATGCCTACGT CGCCAACATT TCTGGAGGGC AACACAGTGG TCTGAGGGGA 1100
AATGTGAACT GATCTGTAAC CAAGCCACAG AGAGAGCTC @1139
SEQ ID NO: 2
LENGTH OF SEQUENCE : 352
TYPE OF SEQUENCE : amino acid
TYPE OF SEQUENCE : polypeptide
SEQUENCE DESCRIPTION
Met Gly Gly Leu Tyr Ser Glu Tyr Leu Asn Pro Glu Lys Val Gln
                5                   10                  15
Glu His Tyr Asn Tyr Thr Lys Glu Thr Leu Asp Met Gln Glu Thr
                20                  25                  30
Pro Ser Arg Lys Val Ala Ser Ala Phe Ile Ile Ile Leu Cys Cys
                35                  40                  45
Ala Ile Val Val Glu Asn Leu Leu Val Leu Ile Ala Val Ala Arg
                50                  55                  60
Asn Ser Lys Phe His Ser Ala Met Tyr Leu Phe Leu Gly Asn Leu
                65                  70                  75
Ala Ala Ser Asp Leu Leu Ala Gly Val Ala Phe Val Ala Asn Thr
                80                  85                  90
Leu Leu Ser Gly Pro Val Thr Leu Ser Leu Thr Pro Leu Gln Trp
                95                  100                 105
Phe Ala Arg Glu Gly Ser Ala Phe Ile Thr Leu Ser Ala Ser Val
                110                 115                 120
Phe Ser Leu Leu Ala Ile Ala Ile Glu Arg Gln Val Ala Ile Ala
                125                 130                 135

Lys Val Lys Leu Tyr Gly Ser Asp Lys Ser Cys Arg Met Leu Met
                140                 145                 150
Leu Ile Gly Ala Ser Trp Leu Ile Ser Leu Ile Leu Gly Gly Leu
                155                 160                 165
Pro Ile Leu Gly Trp Asn Cys Leu Asp His Leu Glu Ala Cys Ser
                170                 175                 180
Thr Val Leu Pro Leu Tyr Ala Lys His Tyr Val Leu Cys Val Val
                185                 190                 195
Thr Ile Phe Ser Val Ile Leu Leu Ala Ile Val Ala Leu Tyr Val
                200                 205                 210
Arg Ile Tyr Phe Val Val Arg Ser Ser His Ala Asp Val Ala Gly
                215                 220                 225
Pro Gln Thr Leu Ala Leu Leu Lys Thr Val Thr Ile Val Leu Gly
                230                 235                 240
Val Phe Ile Ile Cys Trp Leu Pro Ala Phe Ser Ile Leu Leu Leu
                245                 250                 255
Asp Ser Thr Cys Pro Val Arg Ala Cys Pro Val Leu Tyr Lys Ala
                260                 265                 270
His Tyr Phe Phe Ala Phe Ala Thr Leu Asn Ser Leu Leu Asn Pro
                275                 280                 285
Val Ile Tyr Thr Trp Arg Ser Arg Asp Leu Arg Arg Glu Val Leu
                290                 295                 300
Arg Pro Leu Leu Cys Trp Arg Gln Gly LysGly Ala Thr Gly Arg
305 310 315
Arg Gly Gly Asn Pro Gly His Arg Leu Leu Pro Leu Arg Ser Ser
320 325 330

Ser Ser Leu Glu Arg Gly Leu His Met Pro Thr Ser Pro Thr Phe
335 340 345
Leu Glu Gly Asn Thr Val Val
350
SEQ ID NO : 3
LENGTH OF SEQUENCE : 33
TYPE OF SEQUENCE :nucleic acid
STRANDEDNESS : single
TOPOLOGY : linear
TYPE OF SEQUENCE : other nucleic acid synthetic DNA
OTHER CHARACTERISTICS
This nucleic acid is a degenerated mixed primer.
SEQUENCE DESCRIPTION
GGAATTCTGT GYGYSATTGC IITKGAYMGS TAC
SEQ ID NO : 4
LENGTH OF SEQUENCE : 36
TYPE OF SEQUENCE :nucleic acid
STRANDEDNESS : single
TOPOLOGY : linear
TYPE OF SEQUENCE : other nucleic acid synthetic DNA
OTHER CHARACTERISTICS
This nucleic acid is a degenerated mixed primer.
SEQUENCE DESCRIPTION
GGAATTCAKG WAGWAGGGCA GCCAGCAGAI SRYGAA

## Claims

1. A novel peptide having a receptor activity, which is encoded by a DNA containing a base sequence of SEQ ID NO. 1.

2. The novel peptide according to claim 1, wherein said peptide contains a polypeptide having an amino acid sequence of SEQ ID NO. 2 as a major component.

3. A DNA containing a base sequence of SEQ ID NO. 1.
